# EUROPEAN PATENT SPECIFICATION

(11) **EP 1 207 167 B1**
(45) Date of publication and mention of the grant of the patent: **04.10.2006**
(21) Application number: 00942386.4
(22) Date of filing: 29.06.2000
(51) Int. Cl.: C07K 7/00

(54) **PROCESS FOR THE PREPARATION OF LH-RH DERIVATIVES**
VERFAHREN ZUR DARSTELLUNG VON LH-RH-DERIVATEN
PROCEDE DE PREPARATION DE DERIVES DE LH-RH

(30) Priority: 30.06.1999 JP 18630799
(43) Date of publication of application: 22.05.2002
(60) Divisional application: 06002729.9
(73) Proprietor: Takeda Pharmaceutical Company Limited, Osaka 541-0045 (JP)
(72) Inventor: SASAKI, Yasuhiro, Hikari-shi, Yamaguchi 743-0012 (JP); SHIMIZU, Katsuji, Kumage-gun, Yamaguchi 745-0501 (JP)
(74) Representative: Lederer, Franz
(86) International application number: PCT/JP2000/004277
(87) International publication number: WO 2001/002428

(56) References cited:
- EP-A1- 0 910 575
- GB-A- 2 322 077
- US-A- 3 816 386
- US-A- 4 966 851

## Description

### Technical Field

The present invention provides a simple and efficient industrial process for preparing LH-RH derivatives and also provides a process for preparing LH-RH derivatives having a high quality.

### Background Art

As for a process for preparing peptides, which are LH-RH derivatives, or salts thereof, JP 50-59370 A (corresponds to U. S. Patent No. 4,008,209) describes the following process as that for preparing a peptide represented by the general formula

(Pyr)Glu-His-Trp-Ser-Tyr(or Phe)-X-Leu(or Ile or Nle)-Arg-Pro-NH-R

wherein the amino acids indicate L form, unless otherwise specified, X indicates D-Leu, D-Nle, D-Nval, D-Ser, D-Abu, D-Phg, D-Phe or α-Aibu, and R indicates an alkyl group that may have hydroxyl group. 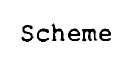 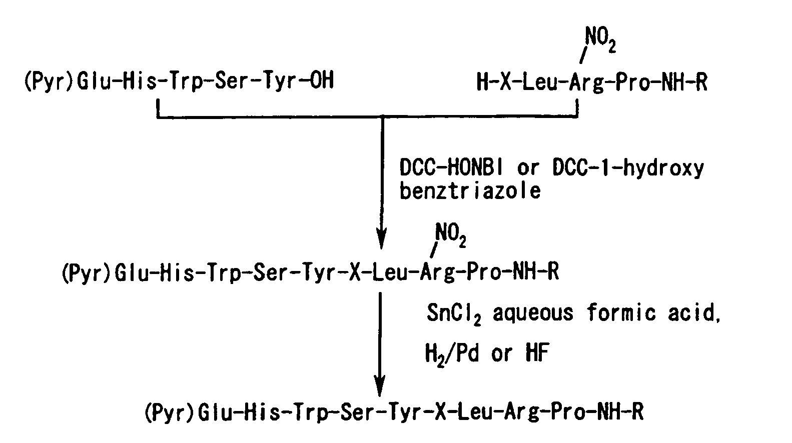 wherein the symbols are as defined above.

Also, JP 51-6926 A (corresponds to U. S. Patent No. 3,997,516) describes a process for preparing a peptide which is characterized by, in the preparation of a peptide having guanidino group, protecting the guanidino group in a starting compound containing guanidino group with a lower alkoxybenzenesulfonyl group or tri-lower alkylbenzenesulfonyl group.

Furthermore, JP 51-100030 A (corresponds to U. S. Patent No. 3,997,516) describes a process for separating/preparing a peptide which is characterized by, in the production of a peptide having guanidino group, protecting the guanidino group in a starting compound containing guanidino group with a lower alkoxy- or tri-lower alkylbenzenesulfonyl group, subjecting the protected compound to peptide condensation and then eliminating said protective group with a halogenosulfonic acid or a lower alkylsulfonic acid or a Lewis acid.

Also, WO 97/48726 describes a process for preparing a peptide represented by the general formula

5-oxo-Pro-R¹-Trp-Ser-R²-R³-R⁴-Arg-Pro-R⁶ (I)

wherein the symbols are as defined below, or a salt thereof which is characterized by reacting a peptide represented by the general formula

5-oxo-Pro-R¹-Trp-Ser-R²-R³-OH (II)

wherein R¹ indicates His, Tyr, Trp or p-NH₂-Phe, R² indicates Tyr or Phe and R³ indicates Gly or an α-D-amino acid residue, each of which may have a substituent, respectively, or a salt thereof with a peptide represented by the general formula

H-R⁴-R⁵-Pro-R⁶ (III)

wherein R⁴ indicates Leu, Ile or Nle, R⁵ indicates Arg that is protected and R⁶ indicates a group represented by the formula Gly-NH-R⁷ (wherein R⁷ indicates hydrogen atom or an alkyl group that may have hydroxyl group) or by the formula NH-R⁸ (wherein R⁸ indicates hydrogen atom, an alkyl group that may have hydroxyl group or ureido group (-NH-CO-NH₂), respectively), or a salt thereof to obtain a peptide represented by the general formula

5-oxo-Pro-R¹-Trp-Ser-R²-R³-R⁴-R⁵-Pro-R⁶ (I')

wherein the symbols are as defined above, or a salt thereof and then subjecting the thus-obtained peptide (I') to a deprotection reaction.

In the prior art, many racemic isomers of constituent amino acids are by-produced during preparation steps of LH-RH derivatives from protected peptides thereof. In the prior art technique, the purification of LH-RH derivatives is carried out by column chromatography using a weakly acidic cation-exchange resin, where multi-step chromatography operations are necessary owing to a poor efficacy of removing racemic isomers, so that it is difficult to produce LH-RH derivatives having a higher quality with a good industrial efficiency. The problems to be solved by the present invention is to provide a process for preparing LH-RH derivatives of a high quality in high yields by an industrially very advantageous method, which utilizes a purification procedure that can suppress racemization of constituent amino acids during the preparation of LH-RH derivatives from protected peptides thereof, can effectively remove racemic isomers and other impurities, and can also carry out the purification step simply and effectively.

### Disclosure of the Invention

As a result of intensive investigations to solve the above-mentioned problems, the present inventors have achieved improvements in the purification step of LH-RH derivatives and improvements in the work-up after the deprotection reaction, and have established a method for suppressing racemization of constitutive amino acids during the preparation and purification steps of LH-RH derivatives as well as a purification method that can effectively remove racemic isomers and other impurities and also can carry out the column treatment step simply and effectively to a great extent without using a step for treatment with an ion-exchange resin, thereby having found a process for preparing LH-RH derivatives of a high quality in high yields. As a result of further intensive investigations on the basis of this finding, the present invention has been completed.

That is, the present invention relates to
(1) a process for preparing a LH-RH derivative which comprises subjecting a solution containing the LH-RH derivative to a step for treatment with a methacrylic synthetic adsorption resin and a step for treatment with an aromatic synthetic adsorption resin;
(2) the process as described in the above (1), wherein the LH-RH derivative is a peptide represented by the formula

   5-oxo-Pro-His-Trp-Ser-Tyr-Y-Leu-Arg-Pro-Z

   wherein Y indicates a residue selected from DLeu, DAla, DTrp, DSer(tBu), D2Nal and DHis(ImBzl), and Z indicates NH-C₂H₅ or Gly-NH₂, respectively, or a salt thereof;
(3) the process as described in the above (1), wherein the LH-RH derivative is a peptide represented by the formula

   5-oxo-Pro-His-Trp-Ser-Tyr-DLeu-Leu-Arg-Pro-NH-C₂H₅

   or its acetate;
(4) the process as described in the above (1), wherein said process comprises using a methacrylic synthetic adsorption resin having a repeating unit represented by the formula 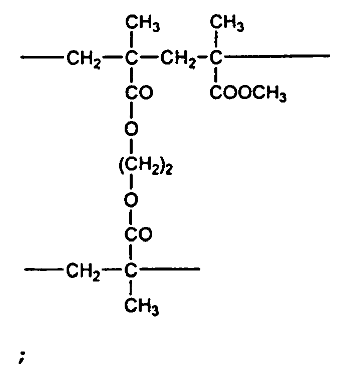
(5) the process as described in the above (1), wherein the aromatic synthetic adsorption resin is a styrene-divinylbenzene synthetic adsorption resin;
(6) the process as described in the above (5), wherein an average particle size of the styrene-divinylbenzene, synthetic adsorption resin is 60 µm to 150 µm;
(7) the process as described in the above (1), wherein said process comprises subjecting a solution containing the LH-RH derivative to the step for treatment with a methacrylic synthetic adsorption resin below 10°C;
(8) the process as described in the above (1), wherein said process comprises subjecting a solution containing the LH-RH derivative to the step for treatment with an aromatic synthetic adsorption resin at 10°C to 20°C;
(9) the process as described in the above (1), wherein said process comprises subjecting a solution containing the LH-RH derivative to the step for treatment with a methacrylic, synthetic adsorption resin, followed by subjecting to the step for treatment with an aromatic, synthetic adsorption resin;
(10) the process as described in the above (1), said process comprises passing a solution containing the LH-RH derivative through a resin in the step for treatment with a methacrylic synthetic adsorption resin and then eluting the LH-RH derivative, which is adsorbed on the resin, with an aqueous solution of acetic acid;
(11) the process as described in the above (10), wherein the concentration of an aqueous solution of acetic acid is 0.01 M to 0.50 M;
(12) the process as described in the above (1), wherein said process comprises passing a solution containing the LH-RH derivative through a resin in the step for treatment with a methacrylic, synthetic adsorption resin, followed by washing with an aqueous solution of ethanol, and then by eluting the LH-RH derivative that is adsorbed on the resin;
(13) the process as described in the above (1), wherein a solution containing the LH-RH derivative is that obtained by subjecting the LH-RH derivative protected with protective group(s) to a deprotection reaction followed by a neutralization reaction below 10°C;
(14) the process as described in the above (1), wherein a solution containing the LH-RH derivative is that obtained by subjecting the LH-RH derivative protected with protective group(s) to a deprotection reaction and then a neutralization reaction below 10°C, followed by subjecting the resulting mixture to extraction of the LH-RH derivative and then concentration of the extract below 25°C;
(15) the process as described in the above (13) or (14), wherein the LH-RH derivative protected with protective group(s) is represented by the formula

   5-oxo-Pro-His-Trp-Ser-Tyr-Y-Leu-Arg(X)-Pro-Z

   wherein X indicates a protective group, Y indicates a residue selected from DLeu, DAla, DTrp, DSer(tBu), D2Nal and DHis(ImBzl) and Z indicates NH-C₂H₅ or Gly-NH₂, respectively;
(16) purified leuprorelin or a salt thereof, wherein the content of total related substances is 1% or less;
(17) purified leuprorelin or a salt thereof, wherein the content of 5-oxo-Pro-D-His-Trp-Ser-Tyr-D-Leu-Leu-Arg-Pro-NH-CH₂-CH₃ or a salt thereof is 0.3% or less.

### Best Mode for Carrying out the Invention

The present invention relates to a process for preparing a LH-RH derivative which comprises subjecting a solution containing the LH-RH derivative to a step for treatment with a methacrylic synthetic adsorption resin and a step for treatment with an aromatic synthetic adsorption resin.

Examples of the LH-RH agonist include peptide LH-RH derivatives possessing LH-RH agonistic activity and salts thereof and there are, for example, peptide LH-RH derivatives possessing LH-RH agonistic activity and salts thereof that are effective against hormone-dependent diseases, particularly sex hormone-dependent cancers (for example, prostatic cancer, uterine cancer, breast cancer and pituitary tumor), prostatic hypertrophy, endometriosis, uterine myoma, precocious puberty, dysmenorrhea, amenorrhea, premenstrual syndrome and polysystic ovary syndrome, as well as for contraception (or against infertility if the post-withdrawal rebound effect is exploited). In addition, they include, for example, LH-RH derivatives and salts thereof, which are effective against benign or malignant tumors that are LH-RH-sensitive though being sex hormone-independent.

As for the above-mentioned salt of the LH-RH derivative, a pharmacologically acceptable salt is preferred, and examples of such a salt, in the case where said LH-RH derivative has a basic group such as amino group, include a salt with an inorganic acid (also, designated as an inorganic free acid) (for example, carbonic acid, bicarbonic acid, hydrochloric acid, sulfuric acid, nitric acid and boric acid), a salt with an organic acid (also, designated as an organic free acid) (for example, succinic acid, acetic acid, propionic acid and trifluoroacetic acid).

Examples of such a salt, in the case where said LH-RH derivative has an acidic group such as carboxyl group, include a salt with an inorganic base (also, designated as an inorganic free base) (for example, an alkali metal such as sodium, potassium, an alkaline earth metal such as calcium, magnesium), a salt with an organic base (also, designated as an organic free base) (for example, an organic amine such as triethylamine, a basic amino acid such as arginine). In addition, said LH-RH derivative may form a metal complex compound (for example, a copper complex or a zinc complex).

As the peptide LH-RH derivative possessing LH-RH agonistic activity, there is, for example, a polypeptide represented by the formula

5-oxo-Pro-R₁-Trp-Ser-R₂-R₃-R₄-Arg-Pro-R₅ (I)

wherein R₁ indicates His, Tyr, Trp or p-NH₂-Phe, R₂ indicates Tyr or Phe and R₃ indicates Gly or a D-amino acid residue that may have a substituent, R₄ indicates Leu, Ile or Nle, R₅ indicates a group represented by the formula Gly-NH-R₆ (wherein R₆ indicates hydrogen atom or an alkyl group that may be substituted) or the formula Gly-NH-R_{6'} (wherein R_{6'} indicates (1) hydrogen atom, (2) an alkyl group that may be substituted with amino group or hydroxyl group) or (3) ureido group (-NH-CO-NH₂).

In the above-mentioned formula (I), the D-amino acid residue indicated by R₃ is exemplified by an α-D-amino acid having up to 11 carbon atoms (for example, D-Leu, Ile, Nle, Val, Nval, Abu, Phe, Phg, Ser, Thr, Met, Ala or Trp), each of which may have 1 to 3 adequate substituents (for example, a C₁₋₄ alkyl group such as methyl, t-butyl, a C₁₋₄ alkoxyl group such as t-butoxy, a C₁₋₄ alkoxycarbonyl group such as t-butoxycarbonyl, a C₆₋₁₀ aryl group such as 2-naphthyl, an indolyl group or an imidazolyl group, which may be substituted with C₁₋₄ alkyl, C₆₋₁₀ aryl or C₆₋₁₀ aryl-C₁₋₄ alkyl, respectively, such as indolyl-3-yl, 2-methylindolyl or benzylimidazol-2-yl). Examples of the substituent of an alkyl group that may be substituted, which is indicated by R_{6'} include hydroxyl or amino. The alkyl group of an alkyl group that may be substituted with amino group or hydroxyl group is exemplified by a C₁₋₄ alkyl group and a C₁₋₃ alkyl group is especially preferred. Examples of a C₁₋₄ alkyl group include methyl, ethyl, propyl, isopropyl, butyl, isobutyl, sec-butyl or tert-butyl. The number of substituents is, for example, 1 to 3, where 1 to 2 substituents are preferable and one substituent is particularly preferable.

More preferably, examples of the peptide LH-RH derivative possessing LH-RH agonistic activity include a physiologically active peptide represented by the formula (II)

5-oxo-Pro-His-Trp-Ser-Tyr-Y-Leu-Arg-Pro-Z

wherein Y indicates a residue selected from DLeu, DAla, DTrp, DSer(tBu), D2Nal and DHis(ImBzl), and Z indicates NH-C₂H₅ or Gly-NH₂, respectively, and a salt thereof. Particularly preferable is such a peptide in which Y is Dleu and Z is NH-C₂H₅ (namely, the peptide represented by 5-oxo-Pro-His-Trp-Ser-Tyr-DLeu-Leu-Arg-Pro-NH-C₂H₅: leuprorelin). As for a salt of the peptide represented by 5-oxo-Pro-His-Trp-Ser-Tyr-DLeu-Leu-Arg-Pro-NH-C₂H₅, its acetate (leuprorelin acetate) is particularly preferred among those exemplified as mentioned above.

The abbreviations used herein for denoting amino acids, peptides, protective groups, etc., in the polypeptides are those according to IUPAC-IUB Commission on Biochemical Nomenclature or conventional codes in this art field, and, also, in the case where an optical isomer may exist with regard to an amino acid, it shall be indicated by the L form, unless otherwise specified.

Examples of the abbreviations are as follows.
- Abu :: Aminobutyric acid
- Aibu :: 2-Aminobutyric acid
- Ala:: Alanine
- Arg:: Arginine
- Gly:: Glycine
- His:: Histidine
- Ile:: Isoleucine
- Leu:: Leucine
- Met:: Methionine
- Nle:: Norleucine
- Nval:: Norvaline
- Phe:: Phenylalanine
- Phg:: Phenylglycine
- Pro:: Proline
- (Pyr)Glu:: Pyroglutamic acid
- Ser:: Serine
- Thr:: Threonine
- Trp:: Tryptophan
- Tyr :: Tyrosine
- Val :: Valine
- D2Nal :: D-3-(2-Naphthyl)alanine residue
- DSer(tBu) :: O-tert-Butyl-D-serine
- DHis(ImBzl) :: N^{im}-Benzyl-D-histidine
- PAM :: Phenylacetamidomethyl
- Boc :: t-Butoxycarbonyl
- Fmoc :: 9-Fluorenylmethyloxycarbonyl
- Cl-Z :: 2-Chlorobenzyloxycarbonyl
- Br-Z :: 2-Bromobenzyloxycarbonyl
- Bzl :: Benzyl
- Cl₂-Bzl :: 2,6-Dichlorobenzyl
- Tos :: p-Toluenesulfonyl
- HONb :: N-Hydroxy-5-norbornene-2,3-dicarboxyimide
- HOBt :: 1-Hydroxybenzotriazole
- HOOBt :: 3-Hydroxy-3,4-dihydro-4-oxo-1,2,3-benzotriazine
- MeBzl :: 4-Methylbenzyl
- Bom :: Benzyloxymethyl
- Bum :: t-Butoxymethyl
- Trt :: Trityl
- DNP :: Dinitrophenyl
- DCC :: N,N'-Dicyclohexylcarbodiimide

Preferable examples of the peptide LH-RH derivative possessing LH-RH agonistic activity include, in addition to the above-mentioned leuprorelin (leuprorelin acetate),

### (1) Goserelin

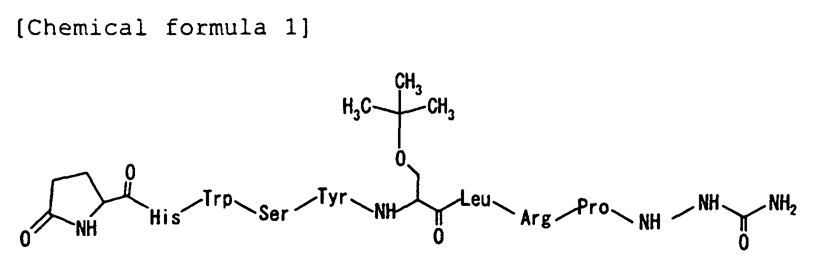

(U.S. Patent No. 4,100,274 and JP 52-136172 A),

### (2) Buserelin

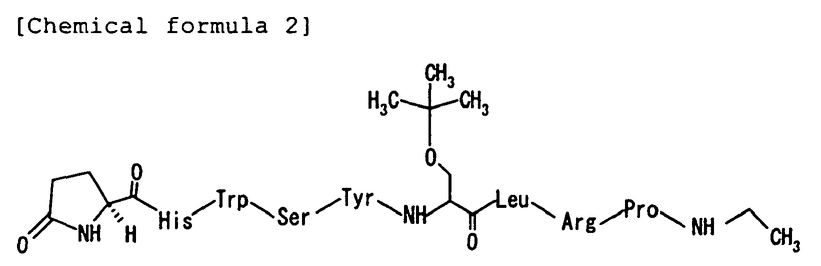

(U.S. Patent No. 4,024,248, German Patent No. 2438352 and JP 51-41359 A),

### (3) Triptorelin

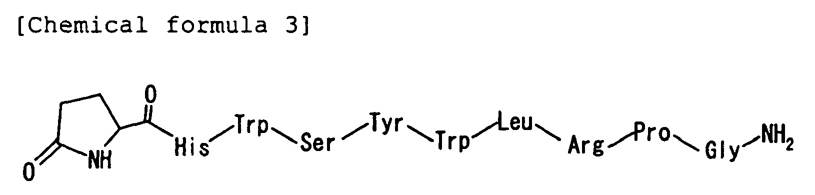

(U.S. Patent No. 4,010,125 and JP 52-31073 A),

### (4) Nafarelin

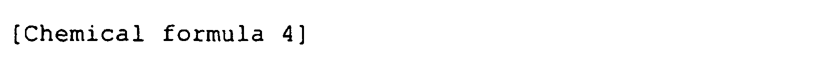

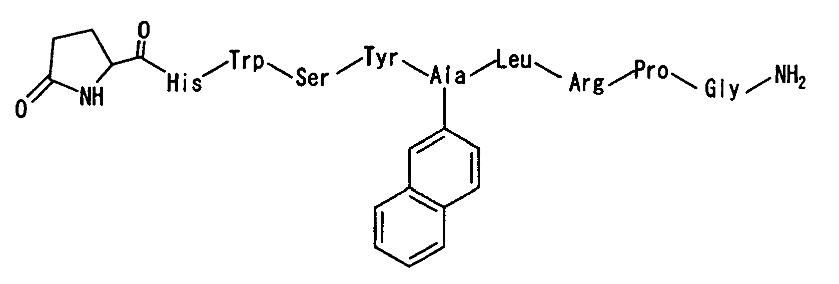

(U.S. Patent No. 4,234,571, JP 55-164663 A, JP 63-264498 A and JP 64-25794 A),

### (5) Historelin

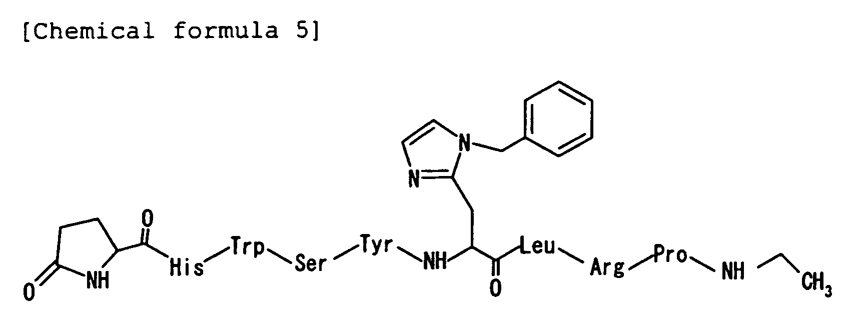

### (6) Deslorelin

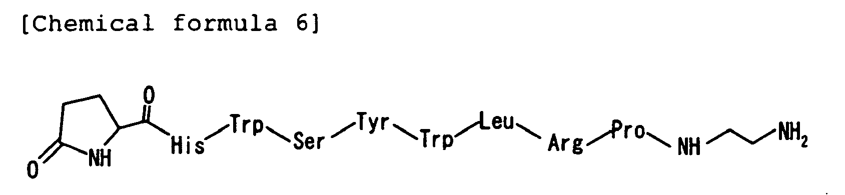

(U.S. Patent No. 4,569,967 and U.S. Patent No. 4,218,439),

### (7) Meterelin

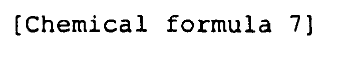

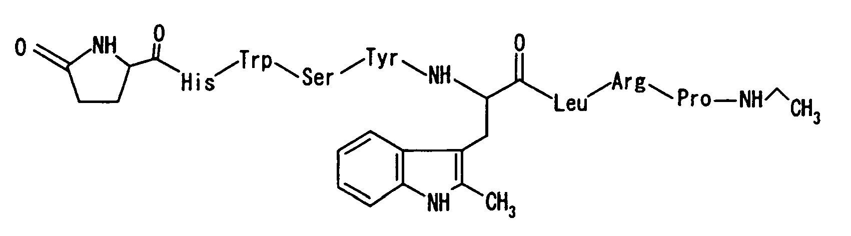

(PCT WO 91/18016),

### (8) Gonadrelin

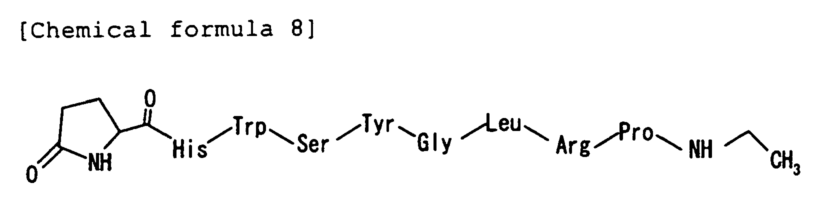

(German Patent No. 2213737) and salts thereof.

In the above-mentioned [Chemical formula 1] to [Chemical formula 8], the amino acid corresponding to R₃ in the afore-mentioned formula (I) is in the D-form.

The methacrylic synthetic adsorption resin means a synthetic adsorption resin of a polymer whose substrate is a methacrylic acid ester, and racemic isomers of the LH-RH derivative can be unexpectedly and effectively removed by subjecting a solution containing the LH-RH derivative to a step for treatment with said resin (particularly by the use of an aromatic, synthetic adsorption resin as described hereinafter in combination) to prepare (purify) the LH-RH derivative.

In addition, racemic isomers of the LH-RH derivative can be effectively removed so that a step for treatment with columns in multi stages, which has been heretofore carried out, can be shortened.

Specific examples of the methacrylic synthetic adsorption resin column include HP 2MG (manufactured by Mitsubishi Chemical Corporation), XAD-7 and XAD-8 (manufactured by Organo Company) (preferably, HP 2MG (manufactured by Mitsubishi Chemical Corporation)), but any one may be used as far as it achieves the object to effectively remove racemic isomers of the LH-RH derivative.

Preferably, the methacrylic synthetic adsorption resin has such particle distribution of the resin that 90% or more of its resin particles have a particle size of 300 µm or more. Also, the methacrylic synthetic adsorption resin having a repeating unit represented by the formula 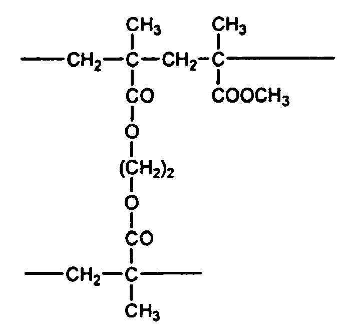 is preferred.

Particularly, in the case where the above-mentioned leuprorelin (leuprorelin acetate) is prepared (purified) with the methacrylic synthetic adsorption resin (preferably, HP 2MG (manufactured by Mitsubishi Chemical Corporation)), there can be very effectively removed a racemic isomer at His adjacent to 5-oxo-Pro in leuprorelin (leuprorelin acetate) (hereinafter, abbreviated as D-His² form), a racemic isomer at Trp adjacent to His (hereinafter, abbreviated as D-Trp³ form) and other highly polar related substances.

The above-mentioned "other highly polar related substances" refer to peptide fragments formed by cleavage of the peptide of leuprorelin, reagents used in the reactions, where specific examples include phenol and the like.

The aromatic synthetic adsorption resin (preferably, a styrene-divinylbenzene synthetic adsorption resin) means a synthetic adsorption resin of a porous polymer, which is prepared by copolymerization of styrene and divinylbenzene, where racemic isomers of the LH-RH derivative can be unexpectedly and effectively removed by subjecting a solution containing the LH-RH derivative to a step for treatment with said resin (particularly by the use of the methacrylic synthetic adsorption resin described above in combination) to prepare (purify) the LH-RH derivative. Specific examples of the aromatic synthetic adsorption resin include HP 20 and HP 21 (manufactured by Mitsubishi Chemical Corporation), HP 20SS and SP 20SS (manufactured by Mitsubishi Chemical Corporation), XAD-2 and XAD-4 (manufactured by Organo Company) (preferably, HP 20SS (manufactured by Mitsubishi Chemical Corporation)), but any one may be used as far as it achieves the object to effectively remove racemic isomers of the LH-RH derivative.

Also, it is preferable to use the aromatic synthetic adsorption resin having a particle size of about 60 µm to about 150 µm.

In addition, it is preferable to use the styrene-divinylbenzene synthetic adsorption resin having such particle distribution of the resin that 15% or less of its resin particles have a particle size of 150 µm or more, 70% or more of its resin particles have a particle size of 63 µm or more to 150 µm or less and 20% or less of its resin particles have a particle size of 63 µm or less.

Particularly, in the case where the above-mentioned leuprorelin (leuprorelin acetate) is prepared (purified) by using the aromatic synthetic adsorption resin (preferably, HP 20SS (manufactured by Mitsubishi Chemical Corporation)), there can be very effectively removed D-His² form and L-Leu⁶ form in leuprorelin (leuprorelin acetate) as well as other highly polar, related substances.

The above-mentioned "other highly polar, related substances" has the same meaning as described above.

In order to achieve the above-mentioned object to "effectively remove racemic isomers, etc. of an LH-RH derivative", it is preferable to employ a step for treatment with the methacrylic synthetic adsorption resin and a step for treatment with the aromatic synthetic adsorption resin in combination. In this case, the order of the step for treatment with the methacrylic synthetic adsorption resin and the step for treatment with the aromatic synthetic adsorption resin is not specified particularly in the process for preparing (purifying) the LH-RH derivative, but it is preferable to prepare (purify) the LH-RH derivative by subjecting it to the step for treatment with the methacrylic synthetic adsorption resin and then to the step for treatment with an aromatic, synthetic adsorption resin.

Hereinafter, there is described in detail the process for preparing the LH-RH derivative which comprises subjecting a solution containing the LH-RH derivative to the step for treatment with the methacrylic synthetic adsorption resin and the step for treatment with the aromatic synthetic adsorption resin.

### (1) Purification step of LH-RH derivative

[1] The LH-RH derivative before purification (hereinafter referred to as the crude LH-RH derivative) is dissolved in a buffer solution to prepare the solution containing the LH-RH derivative.
   Examples of the buffer solution to be used include, though not being particularly limited as far as it does not inhibit absorption of the LH-RH derivative on the synthetic adsorption resin, water (distilled water or deionized water), an aqueous solution of sodium acetate, an aqueous solution of ammonium acetate, an aqueous solution of sodium phosphate, an aqueous solution of ammonium phosphate and an aqueous solution of ammonium chloride (preferably, an aqueous solution of sodium acetate).
   In addition, in order to suppress by-product formation of racemic isomers of the LH-RH derivative, it is preferable to adjust pH of the solution containing the LH-RH derivative at 4 to 6, preferably 4 to 5, by addition of a pH-adjusting agent (for example, acetic acid, phosphoric acid or hydrochloric acid).
   Furthermore, in the case where the LH-RH derivative is the above-mentioned leuprorelin (leuprorelin acetate), it is preferable to control a temperature of the solution containing the LH-RH derivative below 10°C, preferably at 3°C to 7°C, in order to prevent by-product formation of a racemic isomer at Trp adjacent to His in leuprorelin (leuprorelin acetate) (hereinafter, abbreviated as D-Trp³ form) and a racemic isomer at Ser adjacent to Trp in leuprorelin (leuprorelin acetate) (hereinafter, abbreviated as D-Ser⁴ form).
   In addition, in order to prevent by-product formation of a racemic isomer (specifically, D-Trp³ form in the case of the above-mentioned leuprorelin (leuprorelin acetate)) and highly polar related substances, it is preferable to subject the solution containing the LH-RH derivative promptly after the preparation to the next operation (the step for treatment with the methacrylic synthetic adsorption resin).
[2] The solution containing the LH-RH derivative obtained in the above-mentioned item [1] is subjected to the step for treatment with the methacrylic, synthetic adsorption resin.
   Specifically, first, the LH-RH derivative is adsorbed on the resin by passing the solution containing the LH-RH derivative obtained in the above [1] through the methacrylic synthetic adsorption resin and then washed with a buffer solution and/or an aqueous solution of an alcohol (for example, an aqueous solution of ethanol, an aqueous solution of methanol, an aqueous solution of n-propanol or an aqueous solution of isopropanol (preferably, an aqueous solution of ethanol)). Examples of the buffer solution to be used include water (distilled water or deionized water), an aqueous solution of sodium acetate, an aqueous solution of ammonium acetate, an aqueous solution of sodium phosphate, an aqueous solution of ammonium phosphate and an aqueous solution of ammonium chloride (preferably, an aqueous solution of sodium acetate or an aqueous solution of ammonium acetate). Also, the highly polar related substances (specifically, vide supra) can be effectively removed by washing the resin with an aqueous solution of ethanol. In this case, it is preferable that the concentration of the aqueous solution of ethanol is about 0 to 20% by volume, preferably 5 to 15% by volume.
   Next, the LH-RH derivative adsorbed on the resin is eluted with an eluent (for example, an aqueous solution of acetic acid, an aqueous solution of propionic acid, hydrochloric acid or an aqueous solution of phosphoric acid, preferably an aqueous solution of acetic acid). In this case, it is preferable that the concentration of the eluent is 0.01 M to 0.50 M, preferably 0.05 M to 0.20 M, more preferably 0.05 M to about 0.10 M. In addition, it is preferable to keep pH of the eluent at neutral or below, preferably pH 3 to 6. By carrying out the elution under these conditions, it is possible to prevent effectively by-product formation of racemic isomers (specifically, D-Trp³ form and D-Ser⁴ form in the case of the above-mentioned leuprorelin (leuprorelin acetate)).
   Also, it is possible to prevent effectively by-product formation of racemic isomers (specifically, D-Trp³ form and D-Ser⁴ form in the case of the above-mentioned leuprorelin (leuprorelin acetate)) by keeping the operation temperature below 10°C, preferably at 0°C to 10°C, more preferably at 3°C to 7°C during the procedure of subjecting the solution containing the LH-RH derivative obtained in the above [1] to the step for treatment with the methacrylic, synthetic adsorption resin.
   A column packed with the methacrylic, synthetic adsorption resin is employed for the step for treatment with the methacrylic, synthetic adsorption resin.
[3] The solution containing the LH-RH derivative obtained by concentration of the solution containing the LH-RH derivative (the eluate) obtained in the above [2] according to a per se known method is subjected to the step for treatment with the aromatic synthetic adsorption resin.

Specifically, first, the solution containing the LH-RH derivative obtained by concentration of the solution containing the LH-RH derivative (the eluate) obtained in the above [2] according to a per se known method is adsorbed the an aromatic, synthetic adsorption resin and then washed with a buffer solution. Examples of the buffer solution to be used include, though not being particularly limited as far as it does not inhibit the absorption of the LH-RH derivative on the synthetic adsorption resin, water (distilled water or deionized water), an aqueous solution of sodium acetate, an aqueous solution of ammonium acetate, an aqueous solution of sodium phosphate, an aqueous solution of ammonium phosphate and an aqueous solution of ammonium chloride (preferably, an aqueous solution of sodium acetate). In addition, the washing may be carried out by the use of an aqueous solution of an alcohol such as an aqueous solution of ethanol, an aqueous solution of methanol, an aqueous solution of n-propanol or an aqueous solution of isopropanol (preferably, an aqueous solution of ethanol).

Next, the LH-RH derivative is eluted with an eluent (for example, an aqueous solution of an alcohol such as an aqueous solution of ethanol, an aqueous solution of methanol, an aqueous solution of n-propanol or an aqueous solution of isopropanol (preferably, an aqueous solution of ethanol)). In this case, it is preferable that the concentration of the eluent is 10% by volume to 50% by volume, preferably about 15% by volume to 40% by volume. In addition, it is preferable to carry out the elution by dividing in several times (preferably, 2 times), and, by carrying out the elution under these preferable conditions, it is possible to remove more effectively racemic isomers (specifically, a racemic isomer at D-Leu adjacent to Trp in leuprorelin (leuprorelin acetate) (hereinafter, abbreviated as L-Leu⁶ form) and the highly polar related substances (specifically, vide supra). In addition, it is preferable that the eluent contains 0% by volume to 0.1% by volume of acetic acid, preferably 0.005% by volume to 0.01% by volume of acetic acid, and, by carrying out the elution under these preferable conditions, it is possible to prevent effectively by-product formation of racemic isomers (specifically, D-Trp³ form and D-Ser⁴ form in the case of the above-mentioned leuprorelin (leuprorelin acetate)).

Also, in the case where the solution containing the LH-RH derivative obtained by concentration of the solution containing the LH-RH derivative (the eluate) obtained in the above-mentioned item [2] according to a per se known method is subjected to the step for treatment with the aromatic synthetic adsorption resin, the removal of racemic isomers (specifically, a racemic isomer at D-Leu adjacent to Trp in leuprorelin (leuprorelin acetate) (hereinafter, abbreviated as L-Leu⁶ form) and the highly polar, related substances (specifically, vide supra) becomes more effective by keeping the operation temperature below room temperature, preferably at 10°C to 20°C, more preferably at 13°C to 17°C.

A column packed with the aromatic synthetic adsorption resin is employed for the step for treatment with the aromatic synthetic adsorption resin.

Subsequently, the LH-RH derivative having a very high quality can be obtained in high yield by concentration of the thus-obtained solution containing the LH-RH derivative (the eluate) according to a per se known method.

Although the order of step [1] and step [2] in the purification process of the LH-RH derivative may be reversed, the removal and the by-product formation of racemic isomers (specifically, D-Trp³ form and D-Ser⁴ form in the case of the above-mentioned leuprorelin (leuprorelin acetate)) become more effective by carrying out the step for treatment with the methacrylic synthetic adsorption resin, followed by the step for treatment with the aromatic synthetic adsorption resin.

### (2) Treatment step before purification and after deprotection reaction

The process of the present invention relates to that for purifying the LH-RH derivative which comprises subjecting the solution containing the LH-RH derivative to the step for treatment with the methacrylic, synthetic adsorption resin and to the step for treatment with the aromatic synthetic adsorption resin. Also, the present invention relates to a process for preparing the LH-RH derivative having a higher quality by carrying out a step for preparing the solution containing the LH-RH derivative, which is obtained by subjecting the LH-RH derivative protected with protective group(s) to a deprotection reaction, prior to the steps for treatment with the synthetic adsorption resins (a step for treatment prior to the purification) at a low temperature.

Hereinafter, the step for treatment prior to the purification is described in detail.

### [1] Production of crude LH-RH derivative

As described hereinabove, the LH-RH derivative obtained by the process of the present invention is a peptide, so that a crude LH-RH derivative can be synthesized according to a per se known method for peptide synthesis. The method for peptide synthesis may be carried out, for example, either by a solid phase synthesis method or a liquid phase synthesis method. That is, the objective peptide can be produced by condensing a partial peptide or an amino acid, which is capable of constituting the crude LH-RH derivative, with the residual part and, in the case where the product have a protective group, by eliminating the protective group. Examples of a known condensation method and the elimination of the protective group include the methods described in the following 1 to 5.
1. M. Bodansky and M. A. Ondetti, Peptide Synthesis, Interscience Publishers, New York (1996)
2. Schroeder and Luebke, The Peptide, Academic Press, New York (1965)
3. Nobuo Izumiya et al. Bases and Experiments for Peptide Synthesis, Maruzen Kabushiki Kaisha (1975)
4. Haruaki Yajima and Syunpei Sakakibara, Lectureships in Biochemistry Experiments 1, Protein Chemistry IV, 205 (1977)
5. Haruaki Yajima, Ed., Continuation of Development of Drugs, Volume 14, Peptide Synthesis, Hirokawa Shoten

For the synthesis of the crude LH-RH derivative, usually, a commercially available resin for a peptide synthesis can be employed. Examples of such a resin can include a chloromethyl resin, a hydroxymethyl resin, benzhydrylamine resin, an aminomethyl resin, a 4-benzyloxybenzyl alcohol resin, a 4-methylbenzhydrylamine resin, a PAM resin, a 4-hydroxymethylmethylphenylacetamidomethyl resin, a polyacrylamide resin, a 4-(2',4'-dimethoxyphenylhydroxymethyl)phenoxy resin and a 4-(2',4'-dimethoxyphenyl-Fmocaminoethyl)phenoxy resin. By using such a resin, amino acids in which the α-amino group and a functional group at a side chain are appropriately protected, can be condensed on the resin according to the sequence of the objective crude LH-RH derivative by a variety of per se known methods to obtain the LH-RH derivative that is protected with protective groups. After the last reaction, the crude LH-RH derivative peptide is isolated from the resin, and a variety of protective groups are removed at the same time to obtain the objective crude LH-RH derivative.

For condensation of the above-mentioned protected amino acids, a variety of activating reagents for a peptide synthesis can be employed, but carbodiimides are particularly favorable. Examples of carbodiimides to be used include DCC, N,N'-diisopropylcarbodiimide and N-ethyl-N'-(3-dimethylaminopropyl)carbodiimide. In the activation by these reagents, a protected amino acid may be added directly to the resin together with an additive for inhibiting racemization (for example, HOBt, HOOBt or HONb), or a protected amino acid may be activated in advance as a symmetric acid anhydride or HOBt ester or HOOBt ester and then added to the resin.

A solvent to be used for activation of a protected amino acid and condensation to the resin may be adequately selected from solvents known to be usable in condensation reactions of peptides. Examples of the solvent to be used include an acid amide such as N,N-dimethylacetamide or N-methylpyrrolidone, a halogenated hydrocarbon such as methylene chloride or chloroform, an alcohol such as trifluoroethanol, a sulfoxide such as dimethyl sulfoxide, pyridine, an ether such as dioxane or tetrahydrofuran, a nitrile such as acetonitrile or propionitrile, an ester such as methyl acetate or ethyl acetate, and an adequate mixture thereof. The reaction temperature is adequately selected from ranges known to be usable in reactions for forming peptide bonds, and usually is adequately selected from a range of -20°C to 50°C. The activated amino acid derivative is usually used in a 1.5- to 4-fold excess. In the case where condensation is found to be insufficient as a result of a test using ninhydrin reaction, sufficient condensation can be achieved by repeating the condensation reaction without elimination of protective groups. Even in the case where sufficient condensation cannot be achieved by repeating the reaction, influence on the following reactions can be avoided by acetylation of unreacted amino acids with acetic anhydride or acetylimidazole.

Examples of the protective groups to be used for the amino group in the starting materials include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, nitro, formyl, 2-nitrophenylsulfonyl, diphenylphosphinothioyl, Fmoc and p-methoxybenzenesulfonyl.

The carboxyl group may be protected, for example, by an alkyl esterification (for example, the formation of an ester of a straight-chain, branched-chain or cyclic alkyl such as methyl, ethyl, propyl, butyl, t-butyl, cyclopentyl, cyclohexyl, cycloheptyl, cyclooctyl or 2-adamantyl), an aralkyl esterification (for example, esterification to form the benzyl ester, the 4-nitrobenzyl ester, the 4-methoxybenzyl ester, the 4-chlorobenzyl ester or the benzhydryl ester), phenacyl esterification, the benzyloxycarbonyl hydrazide formation, the t-butoxycarbonyl hydrazide formation or the trityl hydrazide formation.

The hydroxyl group of serine can be protected, for example, by esterification or etherification. Examples of the group suitable for this esterification include a lower (C₁₋₆) alkanoyl group such as acetyl group, an aroyl group such as benzoyl group, a group, which can be derived from carbonic acid, such as benzyloxycarbonyl group or ethoxycarbonyl group. In addition, examples of a group suitable for the etherification include benzyl group, tetrahydropyranyl group and t-butyl group.

As for the protective group for phenolic hydroxyl group in tyrosine, for example, Bzl, Cl₂-Bzl, p-nitrobenzyl, Br-Z or t-butyl is used.

As for the protective group for imidazole in histidine, for example, Tos, 4-methoxy-2,3,6-trimethylbenzenesulfonyl, DNP, benzyloxymethyl, Bum, Boc, Trt or Fmoc is used.

Examples of the (crude) LH-RH derivative that is protected with protective group(s) include the derivative in which the α-amino group and a functional group at the side chain of any of the constitutive amino acids in the above-mentioned LH-RH derivative are protected with the above-mentioned protective groups.

Specifically, examples of the LH-RH derivative that is protected with protective groups include a peptide represented by the formula (III)

5-oxo-Pro-His-Trp-Ser-Tyr-Y-Leu-Arg(X)-Pro-Z

wherein X indicates a protective group, Y indicates a residue selected from DLeu, DAla, DTrp, DSer(tBu), D2Nal and DHis(ImBzl) and Z indicates NH-C₂H₅ or Gly-NH₂, respectively, or a salt thereof.

As the protective group represented by X in the formula (III), there can be used Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, nitro, formyl, 2-nitrophenylsulfonyl, diphenylphosphinothioyl, Fmoc and p-methoxybenzenesulfonyl, where p-methoxybenzenesulfonyl are particularly preferable.

Examples of the salt of the peptide represented by the formula (III) include salts similar to those described hereinabove.

In addition, the peptide represented by the formula (III) or a salt thereof can be produced by a per se known method for peptide synthesis as described hereinabove, for example, the peptide can be prepared according to the method described in WO No. 97/48726 or modification thereof.

As the method for removing the protective groups (deprotection reaction), there can be employed, for example, hydrogenolysis in a hydrogen stream in the presence of a catalyst such as Pd-black, Pd-charcoal or the like; an acid treatment with anhydrous hydrogen fluoride, methanesulfonic acid, trifluoromethanesulfonic acid, trifluoroacetic acid or a mixed solution thereof (preferably, methanesulfonic acid); a basic treatment with diisopropylethylamine, triethylamine, piperidine or piperazine; reduction with sodium in liquid ammonia. The above-mentioned deprotection reaction is carried out in general at a temperature of about 40°C or lower, where, by carrying out the deprotection reaction below 25°C, preferably at 5°C to 15°C, more preferably at 8°C to 12°C, the by-product formation of racemic isomers of the LH-RH derivative (specifically, D-His² form in leuprorelin (leuprorelin acetate) in the case of the above-mentioned leuprorelin (leuprorelin acetate)) can be effectively inhibited. In the acid treatment, addition of a cation scavenger such as, for example, anisole, phenol, thioanisole, metacresol, paracresol, dimethyl sulfide, 1,4-butanethiol or 1,2-ethanethiol (preferably, phenol) is effective. Also, 2,4-dinitrophenyl group to be used as the protective group for imidazole in histidine is removed by treatment with thiophenol, and formyl group to be used as the protective group for indole in tryptophan is removed by deprotection by the acid treatment in the presence of the above-mentioned 1,2-ethanethiol or 1,4-butanethiol as well as by an alkaline treatment with a dilute solution of sodium hydroxide or dilute ammonia water.

The reaction time in the above-mentioned deprotection reaction is usually about one hour to 10 hours, preferably 2 hours to 5 hours, more preferably 2 hours to 3 hours.

As for a more specific method for removing the protective groups (deprotection), for example, in the case where the peptide represented by the formula (III) or a salt thereof is subjected to the deprotection reaction, a preferable method is exemplified by an acid treatment with anhydrous hydrogen fluoride, methanesulfonic acid, trifluoromethanesulfonic acid, trifluoroacetic acid or a mixed solution thereof (preferably, methanesulfonic acid). In addition, in this case, it is preferable to add a cation scavenger such as, for example, anisole, phenol, thioanisole, metacresol, paracresol, dimethyl sulfide, 1,4-butanethiol or 1,2-ethanethiol (preferably, phenol). The deprotection reaction is carried out in general at a temperature of 40°C or less, where, by carrying out the deprotection reaction below 25°C, preferably at 5°C to 15°C, more preferably at 8°C to 12°C, the by-product formation of racemic isomers of the LH-RH derivative (specifically, D-His² form in leuprorelin (leuprorelin acetate) in the case of the above-mentioned leuprorelin (leuprorelin acetate)) can be effectively inhibited.

Protection of functional groups which should not participate in the reactions of the starting materials, and the protective groups as well as the elimination of the protective groups, the activation of the functional groups, which participate in the reactions, and the like may be appropriately selected from known groups or known means.

### [2] Work-up step after deprotection reaction

First, the solution containing the LH-RH derivative obtained by the deprotection reaction in the above [1] (the reaction solution) is neutralized. The neutralization solution is selected from known solutions, where, for example, in the case where the deprotection reaction is carried out by acid treatment, an aqueous solution of sodium hydroxide, an aqueous solution of potassium hydroxide, an aqueous solution of sodium carbonate, an aqueous solution of sodium hydrogen carbonate, ammonia water or triethylamine (preferably, an aqueous solution of sodium carbonate) is used, and, in the case where the deprotection reaction is carried out by the basic treatment, hydrochloric acid or acetic acid (preferably, hydrochloric acid) is used.

The neutralization reaction is carried out in general at a temperature of 40°C or less, where, by carrying out the neutralization reaction below 10°C, preferably below 5°C, more preferably at -5°C to 5°C, the by-product formation of racemic isomers of the LH-RH derivative (specifically, D-Trp³ form in leuprorelin (leuprorelin acetate) in the case of the above-mentioned leuprorelin (leuprorelin acetate)) can be effectively inhibited.

Next, after an organic layer is separated from a neutralized mixture, a buffer solution (for example, an aqueous solution of sodium acetate, an aqueous solution of ammonium acetate, an aqueous solution of ammonium chloride, an aqueous solution of sodium phosphate or an aqueous solution of ammonium phosphate (preferably, an aqueous solution of sodium acetate) is added to transfer the LH-RH derivative into an aqueous layer. In addition, it is preferable to adjust pH at 3 to 5, preferably at 3.9 to 4.3.

The aqueous layer is washed with ethyl acetate, methyl acetate, toluene, methylene chloride, or ether (preferably, ethyl acetate) and is concentrated by a per se known method, and pH of the solution is adjusted to 4 to 6, preferably to 4.3 to 4.7 with a pH-adjusting agent such as acetic acid, hydrochloric acid or phosphoric acid (preferably, acetic acid) to obtain the solution containing the LH-RH derivative.

A series of steps comprising obtaining the organic layer from the neutralized mixture up to obtaining the solution containing the LH-RH derivative is carried out in general at a temperature of 40°C or less, where, by carrying out these steps at 0°C to 10°C, preferably at 3°C to 7°C, the by-product formation of racemic isomers of the LH-RH derivative (specifically, D-Trp³ form in leuprorelin (leuprorelin acetate) in the case of the above-mentioned leuprorelin (leuprorelin acetate)) can be effectively inhibited.

The LH-RH derivative to be obtained according to the process of the present invention is the LH-RH derivative having an extremely high quality, in which the amount of impurities (racemic isomers of the LH-RH derivative, highly polar related substances and other impurities) are greatly smaller as compared with those in the LH-RH derivative obtained according to the prior art technique. In addition, in the prior art technique, a step for treatment with a synthetic adsorption resin or a step for treatment with an ion-exchange resin has to be repeated several times, whereas, in the present invention, a sufficient purification can be effectively made by carrying out two times of the step for treatment with the synthetic adsorption resin, whereby the LH-RH derivative can be produced in high yields while shortening the operation times. From this point, the process of the present invention is an extremely advantageous process as an industrial process for preparing the LH-RH derivative, as compared with the prior art technique.

As described hereinabove, the LH-RH derivative having an extremely high quality can be obtained according to the process of the present invention.

"The LH-RH derivative having a high quality" is specifically exemplified by a purified LH-RH derivative (preferably, purified leuprorelin or a salt thereof), in which the content of total related substances is 1% or less (preferably, 0.9% or less, more preferably, 0.8% or less, further preferably, 0.7% or less).

Herein, total related substances mean a total of all impurities that are detected by high-performance liquid chromatography, and the impurities are racemic isomers of the LH-RH derivatives, highly polar related substances and other impurities.

More specifically, for example, in the case where the purified LH-RH derivative is purified leuprorelin or a salt thereof, there are exemplified [1] purified leuprorelin or a salt thereof, in which the content of 5-oxo-Pro-D-His-Trp-Ser-Tyr-D-Leu-Leu-Arg-Pro-NH-CH₂-CH₃ or a salt thereof is 0.3% or less (preferably, 0.25% or less, more preferably, 0.2% or less) and [2] purified leuprorelin or a salt thereof, in which the content of 5-oxo-Pro-His-Trp-D-Ser-Tyr-D-Leu-Leu-Arg-Pro-NH-CH₂-CH₃ or a salt thereof is 0.15% or less (preferably, 0.1% or less, more preferably, 0%).

The above-mentioned purified LH-RH derivatives are of a low toxicity and can be administered to mammals (for example, human, monkey, dog, rat and mouse) as prophylactics or therapeutics of sex hormone-dependent diseases such as prostatic cancer, benign prostatic hypertrophy, endometriosis, uterine myoma, uterine fibroid tumor, precocious puberty, breast cancer, ovarian cancer, cervical carcinoma and the like or Alzheimer's disease.

Also, any of the above-mentioned purified LH-RH derivatives can be administered orally as a tablet that is coated as needed, a capsule, an elixir, a sustained-release preparation or the like, or can be administered parenterally as an injectable preparation such as a sterile solution in water or another pharmaceutically acceptable solution, or in the preparation form of nasal administration such as a solution, a suspension or the like. The above-mentioned preparation can be manufactured by admixing the above-mentioned purified LH-RH derivative with a physiologically acceptable known carrier, a flavoring agent, an excipient, a vehicle, a preservative, a stabilizer and a binding agent in a unit dosage form that is required for a generally acceptable pharmaceutical practice.

Examples of additives that can be used for the admixture in a tablet, a capsule and the like include a binding agent such as gelatin, corn starch, tragacanth or gum arabic, an excipient such as crystalline cellulose, a swelling agent such as corn starch, gelatin or alginic acid, a lubricant such as magnesium stearate, a sweetener such as sucrose, lactose or saccharin, a flavoring agent such as peppermint and Akamono oil or cherry. In the case where the dosage unit form is a capsule, a liquid carrier such as an oil and fat can be further contained in addition to the above-mentioned type of materials. A sterile composition for injection can be formulated according to a conventional pharmaceutical practice that is carried out by dissolving or suspending an active substance, a naturally occurring vegetable oil such as sesame oil or coconut oil, in a vehicle such as water for injection. An aqueous solution for injection to be used is exemplified by physiological saline, an isotonic solution containing glucose and other auxiliary agents (for example, D-sorbitol, D-mannitol, sodium chloride and the like), where an adequate solubilizing agent such as an alcohol (for example, ethanol), a polyalcohol (for example, propylene glycol and polyethylene glycol), a non-ionic, surface active agent (for example, polysorbate 80 (TM) and HCO-50) can be used in combination. An oily liquid to be used is exemplified by sesame oil or soybean oil, where a solubilizing agent such as benzyl benzoate or benzyl alcohol can be used in combination.

Also, the above-mentioned preparations may be compounded with a buffering agent (for example, a phosphate buffer solution or a sodium acetate buffer solution) an analgesic (for example, benzalkonium chloride or procaine hydrochloride), a stabilizer (for example, human serum albumin or polyethylene glycol), a preservative (for example, benzyl alcohol or phenol), an antioxidant and the like. The injectable solution thus prepared usually is filled in adequate ampoules.

A sustained-release preparation comprising the above-mentioned purified LH-RH derivative, can be manufactured according to a per se known method such as, for example, the method described in JP 60-100516 A, JP 62-201816 A, JP 4-321622 A, JP 6-192068 A, JP 9-132524 A, JP 9-221417 A, JP 11-279054 A, WO 99/360099.

The dosage of the above-mentioned purified LH-RH derivative varies widely depending on subject disease, subject animal and the like, but the dosage per once can be appropriately selected, for example, preferably from a range of 0.01 mg to 100 mg/kg body weight for an adult patient of prostatic cancer. More preferably, the dosage can be appropriately selected from a range of 0.05 mg to 50 mg/kg body weight.

The dosage of the sustained-release preparation containing said above-mentioned purified LH-RH derivative, for example, in the case of a sustained-release preparation having drug efficacy for one month, the dosage of the sustained-release preparation per once can be appropriately selected preferably from a range of 0.1 mg to 500 mg/kg weight for an adult patient of prostatic cancer. More preferably, the dosage can be appropriately selected from a range of 0.2 mg to 300 mg/kg body weight.

### Examples

The following examples illustrate the present invention in more detail, but they are not intended to restrict the scope of the present invention.

### Example 1 Preparation of 5-oxo-L-prolyl-L-histidyl-L-tryptophyl-L-seryl-L-tyrosyl-D-leucyl-L-leucyl-L-arginyl-N-ethyl-L-prolinamide (leuprorelin, leuprolide) (1)

To a mixed solution of 748 g of methanesulfonic acid and 56 g of phenol was added 68.35 g (52.92 g as pure leuprolide) of 5-oxo-L-prolyl-L-histidyl-L-tryptophyl-L-seryl-L-tyrosyl-D-leucyl-L-leucyl-N^{m}-p-methoxybenzenesulfonyl-L-arginyl-N-ethyl-L-prolinamide, and the resulting solution was reacted at 8 to 12°C for about 3 hours. The reaction solution was added at -5 to 5°C into a mixed solution of a solution of potassium carbonate (646 g) in water (1896 mL) and 396 mL of ethyl acetate, and the resulting mixture was then stirred at 3 to 7°C for 30 minutes and neutralized. The organic layer was separated, mixed with 2316 mL of a 0.11 M sodium acetate buffer solution (pH: 3.9 to 4.3) and stirred at 3 to 7°C to carry out the extraction. The aqueous layer was adjusted to pH 4.3 to 4.7 with acetic acid and mixed with 372 mL of ethyl acetate, and the resulting layers were then separated. The aqueous layer was washed at 3 to 7°C with 372 mL of ethyl acetate. The aqueous layer was concentrated under reduced pressure below 25°C and then adjusted to pH 4.3 to 4.7 with acetic acid to obtain an aqueous solution (1) of leuprolide.
Yield of leuprolide acetate: 44.26 g (90.9%)
Quality by high-performance liquid chromatography (peak area percentage for leuprolide): D-Trp³ form, not detected; D-Ser⁴ form, not detected; D-His² form, 0.27%; L-Leu⁶ form, 1.14%

### Example 2 Preparation of 5-oxo-L-prolyl-L-histidyl-L-tryptophyl-L-seryl-L-tyrosyl-D-leucyl-L-leucyl-L-arginyl-N-ethyl-L-prolinamide (leuprolide) (2)

Through a column packed with 5500 mL of a methacrylic, synthetic adsorption resin (HP 2MG: Mitsubishi Chemical Corporation) was passed at 3 to 7°C 5600 g of the aqueous solution (1) of leuprolide (leuprolide acetate: 85.46 g). The resulting column was washed by passing successively 11000 mL of a 0.3 M aqueous solution of sodium acetate (pH: 6.3), 13750 mL of a 0.025 M aqueous solution of ammonium acetate and 19250 mL of a 10% by volume, aqueous solution of ethanol at 3 to 7°C. The elution was carried out by passing through 41250 mL of a 0.05 M aqueous solution of acetic acid at 3 to 7°C. Fractions containing leuprolide were collected and concentrated under reduced pressure below 35°C to obtain an aqueous solution (2) of leuprolide.
Yield of leuprolide acetate: 73.77 g (86.3%)
Quality by high-performance liquid chromatography (peak area percentage for leuprolide): D-Trp³ form, 0.11%; D-Ser⁴ form, 0.03%; D-His² form, 0.24%; L-Leu⁶ form, 0.42%.

### Example 3 Preparation of 5-oxo-L-prolyl-L-histidyl-L-tryptophyl-L-seryl-L-tyrosyl-D-leucyl-L-leucyl-L-arginyl-N-ethyl-L-prolinamide (leuprolide) (3)

Through a column packed with 2200 mL of small particles of an aromatic, synthetic adsorption resin (HP 20SS: Mitsubishi Chemical Corporation) was passed at 13 to 17°C 3731 g of the aqueous solution (2) of leuprolide (leuprolide acetate: 73.54 g). The resulting column was washed by passing successively 6600 mL of a 0.1 M aqueous solution of sodium acetate (pH: 6.3), 9900 mL of a 0.01 M aqueous solution of ammonium acetate and 2200 mL of water at 13 to 17°C. The elution was carried out by passing through successively 8800 mL of a 20% by volume, aqueous solution of ethanol and 8800 mL of a 35% by volume, aqueous solution of ethanol at 13 to 17°C. Fractions containing leuprolide were collected and concentrated under reduced pressure below 35°C to obtain an aqueous solution (3) of leuprolide.
Yield of leuprolide acetate: 64.55 g (87.8%)
Quality by high-performance liquid chromatography (peak area percentage for leuprolide): D-Trp³ form, 0.12%; D-Ser⁴ form, 0.04%; D-His² form, 0.18%; L-Leu⁶ form, not detected.

### Example 4 Preparation of 5-oxo-L-prolyl-L-histidyl-L-tryptophyl-L-seryl-L-tyrosyl-D-leucyl-L-leucyl-L-arginyl-N-ethyl-L-prolinamide monoacetate (leuprolide acetate) (4)

Column chromatography of 436.7 g of the aqueous solution (3) of leuprolide (leuprolide acetate: 63.90 g) was carried out by using 2350 mL of cross-linked dextran gel (Sephadex LH-20: Pharmacia) with elution with a 0.005 M aqueous solution of acetic acid. Fractions containing leuprolide were collected and subjected to decolorization with 1.16 g of activated carbon. The activated carbon was filtered, the filtrate was concentrated under reduced pressure below 35°C and the concentrate was subjected to ultrafiltration. The filtrate was lyophilized to obtain 60.38 g of freeze-dried leuprolide acetate.
Yield of leuprolide acetate: 60.02 g (98.9%)
Quality by high-performance liquid chromatography (peak area percentage for leuprolide): D-Trp³ form, 0.11%; D-Ser⁴ form, 0.03%; D-His² form, 0.18%; L-Leu⁶ form, not detected.

### Example 5 Preparation of 5-oxo-L-prolyl-L-histidyl-L-tryptophyl-L-seryl-L-tyrosyl-D-leucyl-L-leucyl-L-arginyl-N-ethyl-L-prolinamide (leuprolide) (3')

Through a column packed with 120 L of small particles of an aromatic, synthetic adsorption resin (HP 20SS: Mitsubishi Chemical Corporation) was passed at 13 to 17°C 200 L of the aqueous solution (2) of leuprolide (leuprolide acetate: 4080 g). The resulting column was washed by passing successively 360 L of a 0.1 mol/L aqueous solution of sodium acetate (pH: 6.3), 540 L of a 0.01 mol/L aqueous solution of ammonium acetate and 120 L of water at 13 to 17°C. The elution was carried out by passing through successively 960 L of a 15% by volume, aqueous solution of ethanol (containing 0.01 W/V% of acetic acid), 480 L of a 20% by volume, aqueous solution of ethanol (containing 0.01 W/V% of acetic acid) and 360 L of a 30% by volume, aqueous solution of ethanol (containing 0.01 W/V% of acetic acid) at 13 to 17°C. Fractions containing leuprolide were collected and concentrated under reduced pressure below 35°C to obtain an aqueous solution of leuprolide.
Yield of leuprolide acetate: 3527 g (86.4%)
Quality by high-performance liquid chromatography (peak area percentage for leuprolide): D-Trp³ form, 0.13%; D-Ser⁴ form, 0.04%; D-His² form, 0.25%; L-Leu⁶ form, not detected.

### Industrial Applicability

According to the process of the present invention, it becomes possible to suppress the by-product formation of impurities such as racemic isomers of LH-RH derivatives and to effectively remove the impurities such as the racemic isomers, so that it becomes possible to produce the LH-RH derivatives having an extremely high quality. Also, according to the process of the present invention, a sufficient purification can be made effectively by carrying out two times of a step for treatment with a synthetic, adsorption resin, whereby the LH-RH derivatives can be produced in high yields effectively by easy operations and without involving any complicated procedure for solid-liquid separation.

## Claims

1. A process for preparing a luteinizing hormone releasing hormone (LH-RH) derivative which comprises subjecting a solution containing the LH-RH derivative to a step for treatment with a methacrylic synthetic adsorption resin and a step for treatment with an aromatic synthetic adsorption resin.

2. The process according to claim 1, wherein the LH-RH derivative is a peptide represented by the formula
5-oxo-Pro-His-Trp-Ser-Tyr-Y-Leu-Arg-Pro-Z
wherein Y indicates a residue selected from DLeu, DAla, DTrp, DSer(tBu), D2Nal and DHis(ImBzl), and Z indicates NH-C₂H₅ or Gly-NH₂, respectively, or a salt thereof.

3. The process according to claim 1, wherein the LH-RH derivative is a peptide represented by the formula
5-oxo-Pro-His-Trp-Ser-Tyr-DLeu-Leu-Arg-Pro-NH-C₂H₅
or its acetate.

4. The process according to claim 1, wherein said process comprises using a methacrylic synthetic adsorption resin having a repeating unit represented by the formula

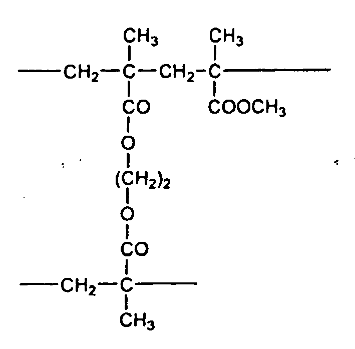

5. The process according to claim 1, wherein the aromatic synthetic adsorption resin is a styrene-divinylbenzene synthetic adsorption resin.

6. The process according to claim 5, wherein an average particle size of the styrene-divinylbenzene, synthetic adsorption resin is 60 µm to 150 µm.

7. The process according to claim 1, wherein said process comprises subjecting a solution containing the LH-RH derivative to the step for treatment with a methacrylic synthetic adsorption resin below 10°C.

8. The process according to claim 1, wherein said process comprises subjecting a solution containing the LH-RH derivative to the step for treatment with an aromatic synthetic adsorption resin at 10°C to 20°C.

9. The process according to claim 1, wherein said process comprises subjecting a solution containing the LH-RH derivative to the step for treatment with a methacrylic, synthetic adsorption resin, followed by subjecting to the step for treatment with an aromatic, synthetic adsorption resin.

10. The process according to claim 1, said process comprises passing a solution containing the LH-RH derivative through a resin in the step for treatment with a methacrylic synthetic adsorption resin and then eluting the LH-RH derivative, which is adsorbed on the resin, with an aqueous solution of acetic acid.

11. The process according to claim 10, wherein the concentration of an aqueous solution of acetic acid is 0.01 M to 0.50 M.

12. The process according to claim 1, wherein said process comprises passing a solution containing the LH-RH derivative through a resin in the step for treatment with a methacrylic, synthetic adsorption resin, followed by washing with an aqueous solution of ethanol, and then by eluting the LH-RH derivative that is adsorbed on the resin.

13. The process according to claim 1, wherein a solution containing the LH-RH derivative is that obtained by subjecting the LH-RH derivative protected with protective group(s) to a deprotection reaction followed by a neutralization reaction below 10°C.

14. The process according to claim 1, wherein a solution containing the LH-RH derivative is that obtained by subjecting the LH-RH derivative protected with protective group(s) to a deprotection reaction and then a neutralization reaction below 10°C, followed by subjecting the resulting mixture to extraction of the LH-RH derivative and then concentration of the extract below 25°C.

15. The process according to claim 13 or 14, wherein the LH-RH derivative protected with protective group(s) is represented by the formula
5-oxo-Pro-His-Trp-Ser-Tyr-Y-Leu-Arg(X)-Pro-Z
wherein X indicates a protective group, Y indicates a residue selected from DLeu, DAla, DTrp, DSer(tBu), D2Nal and DHis(ImBzl) and Z indicates NH-C₂H₅ or Gly-NH₂, respectively.

## Patentansprüche

1. Verfahren zur Herstellung eines Derivats des luteinisierenden Hormon freisetzenden Hormons (LH-RH-Derivat), das beinhaltet, dass eine Lösung, die das LH-RH-Derivat enthält, einer Stufe der Behandlung mit einem synthetischen Methacryladsorptionsharz und einer Stufe der Behandlung mit einem aromatischen synthetischen Adsorptionsharz unterzogen wird.

2. Verfahren nach Anspruch 1, wobei das LH-RH-Derivat ein Peptid der Formel
5-Oxo-Pro-His-Trp-Ser-Tyr-Y-Leu-Arg-Pro-Z
ist, worin Y einen Rest ausgewählt aus DLeu, DAla, DTrp, DSer(tBu), D2Nal und DHis(ImBzl) bedeutet und Z NH-C₂H₅ oder Gly-NH₂ bedeutet, oder ein Salz davon.

3. Verfahren nach Anspruch 1, wobei das LH-RH-Derivat ein Peptid der Formel
5-Oxo-Pro-His-Trp-Ser-Tyr-DLeu-Leu-Arg-Pro-NH-C₂H₅
oder das Acetat davon ist.

4. Verfahren nach Anspruch 1, wobei das Verfahren beinhaltet, dass ein synthetisches Methacryladsorptionsharz mit sich wiederholenden Einheiten der Formel 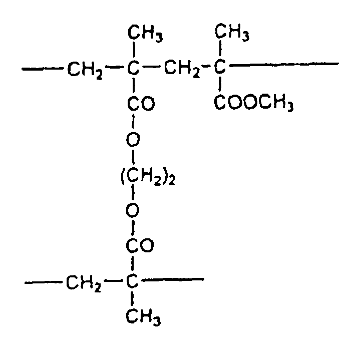 verwendet wird.

5. Verfahren nach Anspruch 1, wobei das aromatische synthetische Adsorptionsharz ein synthetisches Styroldivinylbenzoladsorptionsharz ist.

6. Verfahren nach Anspruch 5, wobei die durchschnittliche Teilchengröße des synthetischen Styroldivinylbenzoladsorptionsharzes 60 µm bis 150 µm ist.

7. Verfahren nach Anspruch 1, wobei das Verfahren beinhaltet, dass eine Lösung, die das LH-RH-Derivat enthält, der Stufe der Behandlung mit einem synthetischen Methacryladsorptionsharz bei einer Temperatur unter 10°C unterzogen wird.

8. Verfahren nach Anspruch 1, wobei das Verfahren beinhaltet, dass eine Lösung, die das LH-RH-Derivat enthält, der Stufe der Behandlung mit einem aromatischen synthetischen Adsorptionsharz bei einer Temperatur von 10 bis 20°C unterzogen wird.

9. Verfahren nach Anspruch 1, wobei das Verfahren beinhaltet, dass eine Lösung, die das LH-RH-Derivat enthält, der Stufe der Behandlung mit einem synthetischen Methacryladsorptionsharz unterzogen wird und anschließend der Stufe der Behandlung mit einem aromatischen synthetischen Adsorptionsharz unterzogen wird.

10. Verfahren nach Anspruch 1, wobei das Verfahren beinhaltet, dass eine Lösung, die das LH-RH-Derivat enthält, in der Stufe der Behandlung mit einem synthetischen Methacryladsorptionsharz über ein Harz geleitet wird und dann das LH-RH-Derivat, das an dem Harz adsorbiert ist, mit einer wässrigen Essigsäurelösung eluiert wird.

11. Verfahren nach Anspruch 10, wobei die Konzentration der wässrigen Essigsäurelösung 0,01 M bis 0,50 M ist.

12. Verfahren nach Anspruch 1, wobei das Verfahren beinhaltet, dass eine Lösung, die das LH-RH-Derivat enthält, in der Stufe der Behandlung mit einem synthetischen Methacryladsorptionsharz über ein Harz geleitet wird und anschließend mit einer wässrigen Lösung von Ethanol gewaschen wird und dann das LH-RH-Derivat, das an dem Harz adsorbiert ist, eluiert wird.

13. Verfahren nach Anspruch 1, wobei eine Lösung, die das LH-RH-Derivat enthält, die ist, die erhalten wird, indem das LH-RH-Derivat, das mit einer oder mehreren Schutzgruppen geschützt ist, einer Reaktion zur Abspaltung der Schutzgruppen und anschließend einer Neutralisierungsreaktion bei einer Temperatur unter 10°C unterzogen wird.

14. Verfahren nach Anspruch 1, wobei eine Lösung, die das LH-RH-Derivat enthält, die ist, die erhalten wird, indem das LH-RH-Derivat, das mit einer oder mehreren Schutzgruppen geschützt ist, einer Reaktion zur Abspaltung der Schutzgruppen und dann einer Neutralisierungsreaktion bei einer Temperatur unter 10°C unterzogen wird und anschließend die entstehende Mischung einer Extraktion des LH-RH-Derivats unterzogen wird und dann der Extrakt bei bei einer Temperatur unter 25°C eingeengt wird.

15. Verfahren nach Anspruch 13 oder 14, wobei das mit einer oder mehreren Schutzgruppen geschützte LH-RH-Derivat durch die Formel
5-Oxo-Pro-His-Trp-Ser-Tyr-Y-Leu-Arg(X)-Pro-Z
dargestellt wird, worin X eine Schutzgruppe bedeutet, Y einen Rest ausgewählt aus DLeu, DAla, DTrp, DSer(tBu), D2Nal und DHis(ImBzl) bedeutet und Z NH-C₂H₅ oder Gly-NH₂ bedeutet.

## Revendications

1. Procédé de préparation d'un dérivé de l'hormone de libération de l'hormone lutéinisante (LH-RH) qui consiste à soumettre une solution comprenant le dérivé de LH-RH à une étape de traitement avec une résine adsorbante synthétique méthacrylique et à une étape de traitement avec une résine adsorbante synthétique aromatique.

2. Procédé selon la revendication 1, dans lequel le dérivé LH-RH est un peptide représenté par la formule
5-oxo-Pro-His-Trp-Ser-Tyr-Y-Leu-Arg-Pro-z
dans laquelle Y indique un résidu choisi parmi DLeu, DAla, DTrp, DSer(tBu), D2Nal et DHis(ImBzl), et Z indique un groupe NH-C₂H₅ ou un groupe Gly-NH₂, respectivement, ou un sel de celui-ci.

3. Procédé selon la revendication 1, dans lequel le dérivé LH-RH est un peptide représenté par la formule
5-oxo-Pro-His-Trp-Ser-Tyr-DLeu-Leu-Arg-Pro-NH-C₂H₅
ou son acétate.

4. Procédé selon la revendication 1, ledit procédé comprenant l'utilisation d'une résine adsorbante synthétique méthacrylique ayant un motif répété représenté par la formule

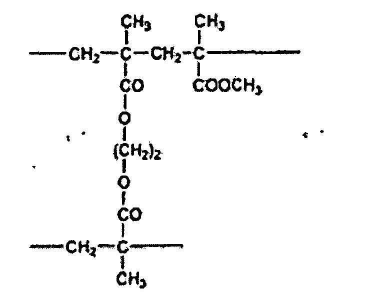

5. Procédé selon la revendication 1, dans lequel la résine adsorbante synthétique aromatique est une résine adsorbante synthétique de styrène-divinylbenzène.

6. Procédé selon la revendication 5, dans lequel une taille particulaire moyenne de la résine adsorbante synthétique de styrène-divinylbenzène est de 60 µm à 150 µm.

7. Procédé selon la revendication 1, ledit procédé consistant à soumettre une solution comprenant le dérivé de LH-RH à une étape de traitement avec une résine adsorbante synthétique méthacrylique à une température inférieure à 10 °C.

8. Procédé selon la revendication 1, ledit procédé consistant à soumettre une solution comprenant le dérivé de LH-RH à une étape de traitement avec une résine adsorbante synthétique aromatique à une température allant de 10 °C à 20 °C.

9. Procédé selon la revendication 1, ledit procédé consistant à soumettre une solution comprenant le dérivé de LH-RH à une étape de traitement avec une résine adsorbante synthétique méthacrylique, suivie d'une étape de traitement avec une résine adsorbante synthétique aromatique.

10. Procédé selon la revendication 1, ledit procédé consistant à passer une solution comprenant le dérivé de LH-RH à travers une résine dans l'étape de traitement avec une résine adsorbante synthétique méthacrylique et ensuite à éluer le dérivé de LH-RH, lequel est adsorbé sur la résine, avec une solution aqueuse d'acide acétique.

11. Procédé selon la revendication 10, dans lequel la concentration d'une solution aqueuse d'acide acétique va de 0,01 M à 0,50 M.

12. Procédé selon la revendication 1, ledit procédé consistant à passer une solution comprenant le dérivé de LH-RH à travers une résine dans l'étape de traitement avec une résine adsorbante synthétique méthacrylique, puis à effectuer un lavage avec une solution aqueuse d'éthanol, et ensuite à éluer le dérivé de LH-RH qui est adsorbé sur la résine.

13. Procédé selon la revendication 1, dans lequel une solution comprenant le dérivé de LH-RH est celle obtenue en soumettant le dérivé de LH-RH protégé par un ou des groupe(s) protecteur(s) à une réaction de déprotection suivie d'une réaction de neutralisation à une température inférieure à 10 °C.

14. Procédé selon la revendication 1, dans lequel une solution comprenant le dérivé de LH-RH est celle obtenue en soumettant le dérivé de LH-RH protégé par un ou des groupe(s) protecteur(s) à une réaction de déprotection et ensuite à une réaction de neutralisation à une température inférieure à 10 °C, suivie par la soumission du mélange résultant à une extraction du dérivé de LH-RH et ensuite à une concentration de l'extrait à une température inférieure à 25 °C.

15. Procédé selon la revendication 13 ou 14, dans lequel le dérivé de LH-RH protégé par un ou des groupe(s) protecteur(s) est représenté par la formule
5-oxo-Pro-His-Trp-Ser-Tyr-Y-Leu-Arg(X)-Pro-z
dans laquelle X indique un groupe protecteur, Y indique un résidu choisi parmi DLeu, DAla, DTrp, DSer(tBu), D2Nal et DHis(ImBzl), et Z indique un groupe NH-C₂H₅ ou un groupe Gly-NH₂, respectivement.
